(19) Europäisches Patentamt
European Patent Office
Office européen des brevets

(11) Publication number: 0 452 067 A2

# (12) EUROPEAN PATENT APPLICATION

(21) Application number: 91303074.8

(22) Date of filing: 08.04.91

(51) Int. Cl.[5]: C07H 3/06, C07H 15/203, C07H 23/00, C12Q 1/40

(30) Priority: 10.04.90 US 507689
14.12.90 US 627941

(43) Date of publication of application:
16.10.91 Bulletin 91/42

(84) Designated Contracting States:
AT BE CH DE FR GB IT LI NL SE

(71) Applicant: GENZYME CORPORATION
One Kendall Square
Cambridge, Massachusetts 02139 (US)

(72) Inventor: Rasmussen, James R.
The Esplanade, 75 Cambridge Parkway E411
Cambridge, Massachusetts 02142 (US)
Inventor: Schena, David
1244 Beacon Street
Brookline, Massachusetts 02146 (US)

(74) Representative: Froud, Clive et al
Elkington and Fife Prospect House 8
Pembroke Road
Sevenoaks, Kent TN13 1XR (GB)

## (54) Oligosaccharide compounds and preparation thereof.

(57) Inter alia, a compound characterised in that it corresponds to the following general formula:

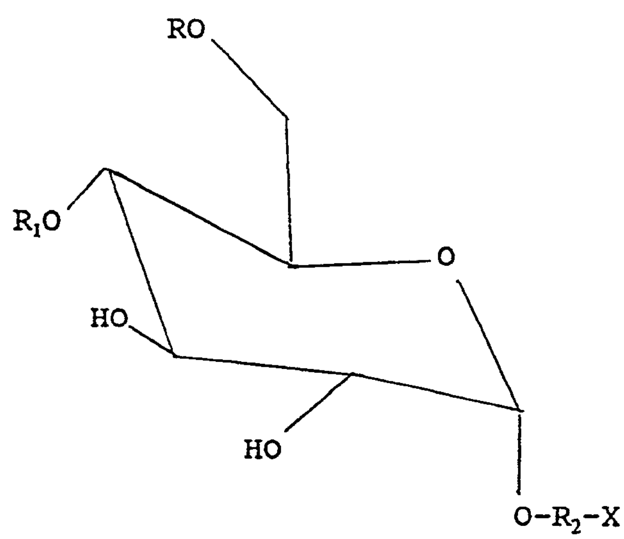

wherein R represents a straight-chained or branched alkyl or aryl substituted silyl radical comprising the formula $R_3R_4R_5Si$-, wherein $R_1$ represents H; $R_3$, $R_4$, $R_5$, if alkyl chains, contain from 1 to 6 carbon atoms each, and, if aryl, are substituted with hydrogen, methyl or methoxy radicals; $R_2$ represents an oligoglucoside residue containing at least two glucose units; and X represents a hydrogen atom or a label which exhibits an optically measurable change upon cleavage of the bond between $R_2$ and X is dosclosed, as it the preparation thereof.

There silyl blocked oligosaccharide compounds are useful in assays for detecting α-amylase. The most preferred compound comprises 6-0-thexyldimethylsilyl-4-nitrophenyl-α-D-maltoheptaoside.

EP 0 452 067 A2

The present invention is concerned with new oligosaccharide compounds, with their preparation, and with their use as improved substrates for the assay of α-amylase.

The measurement of α-amylase in urine and serum is widely performed in the diagnosis of pancreatic disorders. Traditionally, such assays have employed oligosaccharide α-amylase substrates which are cleaved into smaller fragments by α-amylase, in conjunction with an exo-enzyme, e.g., α-glucosidase, β-glucosidase or glucoamylase.

Blair in United States Patent No. 4,649,108 describes an assay for α-amylase which employs oligosaccharides of chain length at least three glucose units, with a chromophore on the reducing end, and with "a blocking" substituent (e.g. carboxylic acid esters (such as acetyl or benzoyl); phosphate esters; sulfonate esters (such as toluenesolfonyl or methanesulfonyl); ethers (such as benzyl, silyl, and triphenylmethyl); and monosaccharides other than α-1,4 linked glucose) on the non-reducing end with 4,6-O-benzylidene being the preferred blocked substrate. The "blocking" substituent imparts resistance to cleavage by the exo-enzymes employed in the assay.

Rauscher et al. in United States Patent No, 4,709,020 describes compounds and processes for their preparation which are related to and included in Blair United States Patent No. 4,649,108. Rauscher et al. describe advantages of such compounds (e.g. 4,6-0-benzylidene-4-nitropheny-maltoheptaoside) similar to those described by Blair in an α-amylase determination method.

Omichl and Ikenaka (1983) J. Blochem. 1055 describe fluorogenic oligosaccharide substrates for α-amylase which are substituted with a 2-pyridylamino group at C-6 of the non-reducing terminal glucose. This compound is resistant to cleavage by exoglucosidases. It does not, however, carry a chromophore (e.g. p-nitrophenol) on the reducing end, and its detection in on amylase assay requires analysis by HPLC, a disadvantage preferably avoided.

Satomura et al. (1988) in Carbohydrate Res. 107 describes a p-nitrophenyl-α-maltopentaoside having a benzyl group on 0-6 of the non-reducing terminal glucose. This compound is suitable for use as a substrate in α-amylase determination (Satomura et al. (1988) Clin, Chem. Acta 315) whereby the benzyl substituent blocks the action of exoglucosidases. The synthesis of this blocked substrate, however, involves highly dangerous, explosive and toxic reagents (e.g. borane dimethylamine), a severe disadvantage also preferably avoided.

Driscoll et al. in United States Patent No. 4,102,747 describe on assay employing oligosaccharides having a chain length of 4-10 glucose units, with a chromophore (p-nitrophenol or "PNP") on the reducing end. The chain is resistant to cleavage by α-glucosidase, and cleavage by α-amylase produces "smaller fragments which are acted upon by α-glycosidase....to liberate p-nitrophenol."

Marshall et al. (1977) Clin. Chimica Acta 277 describe "modified amylaceous polysaccharides containing blockages to the action of glucoamylase. Such blockages to exo-enzyme action are conveniently introduced....by limited periodate oxidation...or by substitution monosaccharide residues."

In general, the present invention features a series of novel "blocked" oligosaccharide substrates with the general formula:

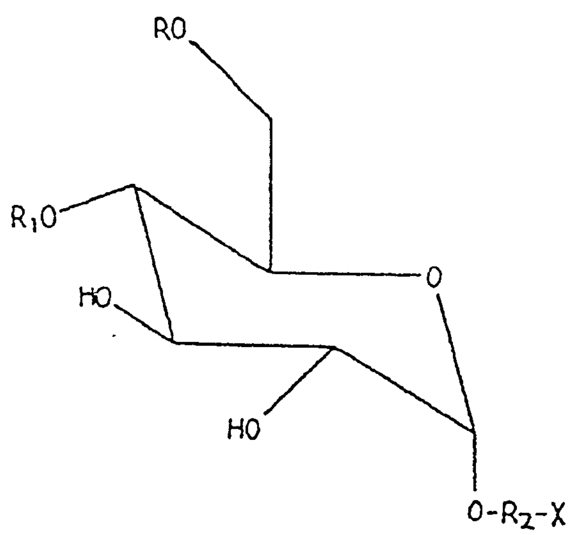

in which R represents a straight-chained or branched alkyl- or aryl- substituted silyl radical with the formula $R_3R_4R_5Si$-; $R_1$ represents H; or $R_1$ and R, independently of each other, each represent a straight-chained or branched alkyl- or aryl- substituted silyl radical, or R and $R_1$ together from a silyl bridge in which the silicon is

disubstituted with either straight-chained or branched alkyl or aryl moieties; $R_2$ represents an oligossacharide residue containing at least two glucose units; and X is a hydrogen atom or a label which exhibits an optically measurable change upon cleavage of the glycosidic bond between $R_2$ and X. The most preferred embodiments of the present invention comprise 6-0-thexyldimethylsilyl-4-nitrophenyl-α-D-maltoheptaoside and 6-0-thexyldimethylsilyl-4-nitrophenyl-α-D-maltoheptaoside.

We have surprisingly discovered that the compounds of the present invention, even in the presence of α-glucosidase, do not undergo any changes and, therefore even after extended periods of time, provide correct α-amylase values. We have further, unexpectedly found that the compounds according to the present invention provide distinct advantages over previously described oligosaccharide substrates, particularly those of Rauscher et al. in United States Patent No. 4,709,020 and Blair in U.S. Patent No. 4,649,108. These advantages derive from improved V/K values with α-amylase, and are manisfestered in part in an improved sensivity to α-amylase, thereby allowing for either use of less substrate per assay, or increased α-amylase detection sensivity. We have further discovered that the compounds, according to the present invention display a unique cleavage pattern when exposed to the endo-enzyme α-amylase, described in detail below.

Referring to the subsequent Tables and the accompanying Figure:

Table 1 presents data describing the binding and kinetic parameters of the compounds according to the present invention with α-amylase.

Table 2 presents data describing the cleavage pattern of the compounds according to the present invention with α-amylase.

The Figure depicts the reaction sequence described in Example 7.

The data in Table 1 illustrate the differences in kinetic and binding values with α-amylase observed among the silyoligosaccharides of this invention and conventional 4,6-0- benzylidene derivates. For example, the binding constant (Km) for the 6-0-t-butyldimethylsilyl-4-nitrophenyl-α-D-maltoheptaoside (silyl-$G_7$-PNP), is 8l uM, compared to a value of 92 uM for the conventional 4,6-0-benzylidene derivative (Bz-$G_7$-PNP). The silyl-$G_7$-PNP is also turned over with a Vmax of $1.15x10^{-2}$ uMoles min $^{-1}$, or 46% faster than the Bz-$G_7$-PNP. The silyl-$G_5$-PNP embodiment of the present invention displays a Km of 87 uM, compared to 183 uM for the conventional Bz-$G_5$-PNP, with a final V/K 19% greater than that of the Bz-$G_5$-PNP. The improved Km and Vmax values for the silyl-$G_7$-PNP derivative indicate that a significant advantage is to be obtained in an assay for α-amylase utilizing this embodiment, whereby (i) less substrate can be used, and (ii) an increased sensitivity for α-amylase can be obtained.

The data presented in Table 2 further illustrate the differences in reactivity with α-amylase of the silyl derivatives of this invention compared with the traditional benzylidene derivatives. These differences are clearly manisfested in the cleavage patterns observed during hydrolysis with both pancreatic and salivary α-amylase. For example, the silyl-$G_7$ derivative is cleaved primarily between glucosis 3 and 4 (66 - 67%), while the benzylidene-$G_7$ is cleaved between glucoses 2 and 3 (63 - 74%). The silyl-$G_5$ derivatives are cleaved almost equally between glucose units 1-2 and 2-3 (40 - 60% each of pancreatic and salivary α-amylase), while the benzylidene-$G_5$ derivative is cleaved primarily between glucose 3 and 4 (78 - 84%). These data support our belief that the silyl oligosaccharides of the present invention appear to α-amylase as fundamentally and surprisingly different substrates compared with the conventional 4,6-0-benzylidene derivatives and therefore do not behave as equivalents.

Other examples of alkyl substituents for each $R_3$, $R_4$, and $R_5$ in the silyl radical include methyl, ethyl, n-propyl, isopropyl, n-butyl, n-pentyl, its isomers, n-hexyl, its isomers, as well as the cyclohexyl radical. Examples of aryl substituents for each of $R_3$, $R_4$, and $R_5$ in the silyl radical include individually phenyl, o, p, or m-tolyl, o, p, or m-methoxyphenyl, and each of the di- and tri-methyl substituted phenyl isomers. Preferred compounds according to the present invention are those in which $R_1$ is H and R is an alkyl-substituted silyl radical, with thexyldimethylsilyl being particularly preferred.

Of the possible oligoglucoside residues comprising $R_2$, those which contain 3, 4 and 6 glucose units are preferred because these substrates present preferred characteristics to the α-amylase to be measured in the assay.

Preferred labels, X, are 4-nitrophenol, 2-nitrophenol, 2-chloro-4-nitrophenol (chromophores), coumarin derivatives such as 4-methylumbelliferone (a fluorophore), and luciferin (a chemiluminescent substitutent), with 4-nitrophenol and 2-chloro-4-nitrophenol being particularly preferred.

The preparation of the compounds, according to the present invention, can advantageously begin with oligoglucosides containing any of a number of blocking groups on the non-reducing terminal glucose C-6 or C-4/C-6 hydroxyl(s) as outlined schematically below. A 4,6-0-benzylidene blocking group is preferred as the starting material. The oligoglucoside also may optionally carry a terminal optically-determinable group X, as identified above.

The free hydroxyl groups are first protected to remove the reactive proton, for example via conversation

to acyl derivatives via treatment with either an acid chloride or acid anhydride.

Suitable acyl moieties include acethyl, proplonyl, butanoyl, pentanoyl, hexanoyl and benzoyl, with acetyl being most preferred. The most preferred acylating agent is acetic anhydride.

The 4,6-0-blocking group, particularly benzylidene, is next removed by mild acid hydrolysis such that the acyl groups and reducing terminus chromophore X are not disturbed. Any of a number of standard acids known to those skilled in the art may be used, with acetic acid being ideally preferred.

The free C-6 hydroxyl, and if desired C-4 hydroxyl, is next converted to its silyl ether derivative via reaction with an active silylating agent. Any of the silylating agents and reaction systems known to those skilled in the art may be used.

Examples of silylating reagents which can be used include, but are not limited to, chlorotrimethylsilane, bis-trimethylsilylacetamide, chlorothexyldimethylsilane, chlorotriphenylsilane and dichorodiphenysilane. Of these, chorothexyldimethylsilane is most preferred.

Finally, the acyl groups are removed via basic hydrolysis. Any of the standard methods for ester hydrolysis known to those skilled in the art may be used, with sodium methoxide in general being preferred. We have discovered that the conditions for removal of the acyl groups in this step are important in defining the yield and purity of the final product. In particular the molar ratio of sodium methoxide to acyl groups and the concentration of the peracyl compound are important in obtaining a successful outcome to the reaction. We found that a molar ratio of at least 0.05 equivalents, up to 2.5 equivalents, of methoxide peracyl group are required, with a 0.1:1 methoxide equivalents:acyl group ratio being ideally preferred. The peracyl silyl compound should be used at a concentration of between 5 and 50 mM, with a concentration of 30 mM being most preferred.

The following examples are given for the purpose of illustrating the present invention and are not be deemed as limiting.

EXAMPLE 1 Process for the preparation of peracetylated 4,6-0-benzylidene-4-nitrophenyl-α-D-maltoheptaoside ("1").

A solution of p-nitrophenyl-4,6-0-benzylidene-α-D-Maltoheptaoside (5 g, 3.65 mmol) and dimethylaminopyridine (440 mg, 3.5 mmol) in 75 mL of pyridine was cooled to 4°C. To this solution was added dropwise, acetic anhydride (30 ml). After stirring overnight, the resulting light brown solution was slowly poured into rapidly stirred ice water (1 1), whereupon the product precipitated as a white solid. The solid was collected and dried, yielding peracetate, "1", (8.02 g, 92%). Thin layer chromatography revelated: $R_f$=0.5 ($CHC1_3$:MeOH, 15:1). pmr (ppm) ($CD1C_3$): ζ 8.25 (d,2H);7.28 (d,2H), 7.3 - 7.5 (m,5H), 2.2 - 1.9 (m of s, acetates).

EXAMPLE 2 Process for the preparation of peracetylated 4,6-dihydroxy-4-nitrophenyl-α-D-maltoheptaoside ("2").

A solution of "1" prepared pursuant to the procedure of Example 1,(8 g, 3.57 mmol) in 440 mL of acetic acid (80%), was heated to 40-45°C overnight. The reaction mixture was poured into 1.5 l of ice water whereupon the product the precipitated as a white solid. The solid, "2", was collected and dried, yielding 6.43 g (83%) of "2". Thin layer chromatography revealed: $R_f$= 0($CHCl_3$:MeOH, 30:l). pmr(ppm)($CDCl_3$): ζ8.25, (d,2H), 7.25 (d,2H), 2.2 - 1.9(m of s, acetates).

EXAMPLE 3 Process for the preparation of peracetylated 6-0-t-butyl-dimethylsilyl-4-nitrophenyl-α-D-maltoheptaoside ("3").

A solution of "2" prepared pursuant to the procedure of Example 2, (6.4 g, 2.98 mmol), dimethylaminopyridine (0.150 g, 1.2 mmol) and t-butyldimethylsilyl chloride (2.5g, 16.6 mmol) in 50 mL DMF was stirred for 3 hours at room temperature. The resulting solution was poured into 10 mL $H_2O$ whereupon the product precipitated as a white solid. The solid was collected and dried, yielding 6.6 g (99%) of "3". Thin layer chromatography revealed:$R_f$=6.0 ($CHCl_3$:MeOH, 15:1). pmr (ppm) ($CDCl_3$):ζ 8.25 (d,2H), 7.25 (d,2H), 2.2 - 1.9 (m of s, acetates), 0.9 (s,9H), 0.1 (s,6H).

EXAMPLE 4 Process for the preparation of 6-0-t-butyldimethylsllyl-4-nitrophenyl-α-D-maltoheptaoside ("4").

A solution of "3" prepared pursuant to the procedure of Example 3, (6.6 g, 2.96 mmol) in 20 mL MeOH was combined with 2mL of 3 M sodium methoxside in MeOH (freshly prepared from Na metal). The resulting solution was stirred for 1 hour and the reaction was stopped by the addition of 0.2 mL acetic acid. The solvent was removed in vacuo to yield a yellow solid (5.0 g). The yellow solid was further purified by HPLC (column XK-50,

15 x 60 cm, packed with TSK HW 40S resin, buffer: water, flow rate 1.5 mL/min, 25 mL/fraction, detection at 280 nm). Fractions 40-58 were pooled, concentrated by ultrafiltration (500 MW cutoff), and lyophilized to yield 3.3 g (80%) of pure "4". pmr (ppm) ($D_2O$): g 8.2 (d,2H), 7.25 (d,2H), 0.8 (s,9H). 0.05 (s,6H), HPLC: retention time 10.7 min., purity: 97 percent by integration (AX-5 column; acetonitrile/$H_2O$ 72:28 v/v, 1.0 mL/min, detection at 305 nm).

EXAMPLE 5 Process for the preparation of 6-0-t-butyldimethyl-silyl-4-nitrophenyl-α-D-maltopentaoside ("5").

The synthesis of "5" was carried out pursuant to the procedures of Examples 1-4 using instead, p-nitrophenyl-4,6-0-benzylidene-α-D-maltopentaoside as the starting material. This sequence began with 1.0 gram (1.57 mmol) of 4,6-0-benzylidene-α-D-maltopentaoside and yielded 0.28 gm of the desired "5" (27% overall yield) following purification pursuant to the procedure of Example 4. pmr (ppm) ($D_20$): ζ 8.2 (d,2H), 7.25 (d,2H), 0.8 (s,9H), 0.0 (s,6H). HPLC: retention time 5.07 min., purity: 94 percent by integration (column and conditions pursuant to those described in Example 4).

EXAMPLE 6 Determination of kinetic and binding values of α-amylase substrates.

All reactions were carried out in 50 mM PIPES buffer (pH 7.0) containing 50 mM NaCl, 5 mM $CaCl_2$, 10 units glucoamylase and 25 units maltase at 27°C. Each substrate concentration was run in duplicate and at least eight concentrations were examined for each substrate. Data from the reaction progress curves were fit to the Michaelis-Menten equation using Marquardt analysis. The Km values were corrected for contaminating unblocked substrate. The values V and K for the $G_7$ substrates are the average of two experiments. The error in the kinetic parameters from each experiment was less than 5% from the average reported. Data are summarized in Table 1.

EXAMPLE 7 Preparation of para-nitrophenyl 6-0-thexyldimethylsilyl-α-maltoheptaoside.

A solution of "2" (Example 2) (21.22 g, 10.05 mmol), dimethylaminopyridine (3.06 g, 25.1 mmol), and thexyldimethylsilyl chloride (1,1,2-trimethylpropylsilyl chloride) (4.49 g, 25.1 mmol) in 212 mL of dimethylformamede was stirred for 18 hours at room temperature. The resulting solution was poured in 750 mL of ice water containing 250 g of crushed ice and the precipitated product collected and dried to yield 20.3 g of monosilylated, partially acethylated product (tlc $R_f$ = 0.42; $CH_2Cl_2$ = MeOH/15:1).

The monosilylated, partially acetylated maltoheptaoside (20.3 g, 9 mmol) was dissolved in 203 mL of methanol and combined with 11.2 mL of 1.68M sodium methoxide in methanol. After one hour 5 mL of acetic acid was added and the solvent removed under vacuum to yield a yellow solid (15 g). The crude product was purified by passage through a HW 40S TSK column (5 x 30 cm) using water as the mobile phase followed by passage through a column (2.5 x 15 cm) containing XAD-7 resin. The absorbed product was eluted with 60 percent aqueous methanol. The product fraction was concentrated in vacuo to remove the methanol and then lyophilized to yield 6.3 g of the desired product. Analysis of the purified product by HPLC (AX-5 column with isocratic elution with $CH_3CN/H_20$:72/28 (v/v) at a flow rate of 1 mL/min., detection at 305 nM) indicated a purity of approximately 97%.

TABLE 1

| SUBSTRATE (a) | Km (uM) | Vmax (uMoles $min^{-1}$) | $(V/K)^{rel}$ |
|---|---|---|---|
| 1 | 81 | $1.15 \times 10^{-2}$ | 1.66 |
| 2 | 92 | $7.86 \times 10^{-3}$ | 1 |
| 3 | 87 | $5.07 \times 10^{-3}$ | 0.68 |
| 4 | 183 | $8.84 \times 10^{-3}$ | 0.57 |

Key: [a]Substrate Identity:

1 = 6-0-t-butyldimethylsilyl-4-nitrophenyl-α-D-maltoheptaoside

2 = 4,6-0-benzylidene-4-nitrophenyl-α-D-maltoheptaoside

3 = 6-0-t-butyldimethylsilyl-4-nitrophenyl-α-D-maltopentaoside

4 = 4,6-0-benzylidene-4-nitrophenyl-α-D-maltopentaoside

TABLE 2

Cleavage Position (% of total)

| Substrate[a] | G 1/2 | G 2/3 | G 3/4 | G 4/5 | G 5/6 | G 6/7 | Total Hydrolysis (%) |
|---|---|---|---|---|---|---|---|
| Salivary Amylase | | | | | | | |
| 1 (Silyl-$G_7$) | 0.2 | 6.0 | 67.2 | 26.5 | - | - | 24.8 |
| 2 (Bz-$G_7$) | 1.9 | 74.0 | 11.9 | b | 12.2 | - | 15.4 |
| 3 (Silyl-$G_5$) | 44.B | 55.1 | - | - | N/A | N/A | 1.3 |
| 4 (Bz-$G_5$) | - | 15.6 | 84.4 | - | N/A | N/A | 8.3 |

| | | | | | | | | |
|---|---|---|---|---|---|---|---|---|
| Pancreatic Amylase | | | | | | | | |
| 1 (Silyl-$G_7$) | 1.1 | 13.3 | 65.6 | 19.9 | - | - | | 69.9 |
| 2 (Bz-$G_7$) | 4.0 | 63.1 | 18.9 | b | 13.6 | - | | 15.1 |
| 3 (Silyl-$G_5$) | 59.4 | 40.6 | - | - | N/A | N/A | | 31.6 |
| 4 (Bz-$G_5$) | 2.5 | 19.4 | 78.1 | - | N/A | N/A | | 42.9 |

Key: [a]Substrate Identity:

1 = 6-O-t-butyldimethylsilyl-4-nitrophenyl-α-D-maltoheptaoside

2 = 4,6-O-benzylidene-4-nitrophenyl-α-D-maltoheptaoside

3 = 6-O-t-butyldimethylsilyl-4-nitrophenyl-α-D-maltopentaoside

4 = 4,6-O-benzylidene-4-nitrophenyl-α-D-maltopentaoside

## Claims

1. A compound characterised in that it corresponds to the following general formula:

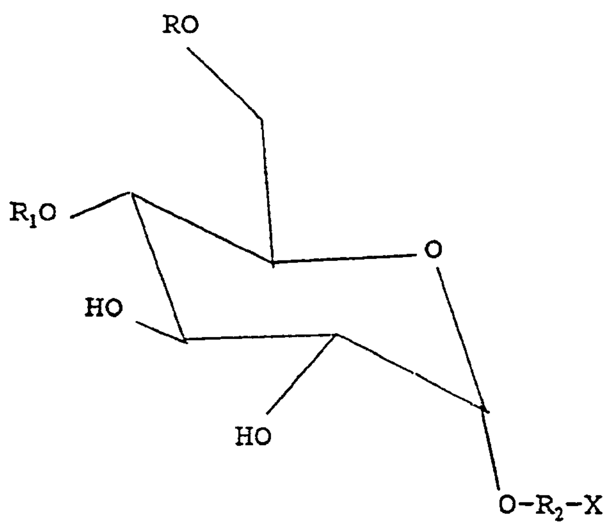

wherein R represents a straight-chained or branched alkyl or aryl substituted silyl radical comprising the formula $R_3R_4R_5Si$-, wherein $R_1$ represents H; $R_3$, $R_4$, $R_5$, if alkyl chains, contain from 1 to 6 carbon atoms each, and, if aryl, are substituted with hydrogen, methyl or methoxy radicals; $R_2$ represents an oligoglucoside residue containing at least two glucose units; and X represents a hydrogen atom or a label which exhibits an optically measurable change upon cleavage of the bond between $R_2$ and X.

2. A compound as claimed in claim 1 wherein X represents nitrophenyl or 2-chloro-4-nitrophenyl or 4-methylumbelliferone or luciferin.

3. A compound as claimed in claim 1 or claim 2 wherein R represents a silyl radical tri-substituted with alkyl radicals having from 1 to 6 carbon atoms per alkyl radical.

4. A compound as claimed in any of claims 1 to 3 wherein the silyl radical is substituted with alkyl radicals selected from methyl, ethyl, n-propyl, isopropyl, n-butyl, isobutyl, t-butyl, n-pentyl and isomers thereof, n-hexyl and isomers thereof, thexyldimiethyl and cyclohexyl; preferably thexyldimethylsilyl; or wherein the silyl radical is substituted with aryl radicals selected from phenyl, o-, p- or m-tolyl, o-, p- or m-methoxyphenyl, and the various di- and tri-methyl-substituted phenyl isomers.

5. A compound as claimed in any of claims 1 to 4 wherein $R_2$ contains 3, 4 or 6 glucose units.

6. A compound as claimed in claim 1 wherein it is 6-0-thexyldimethylsilyl-4-nitrophenyl-α-D-maltoheptaoside or 6-0-thexyldimethylsilyl-4-nitrophenyl-α-D-maltopentaoside.

7. A process for the preparation of a compound as claimed in claim 1 characterised in that it comprises:
(a) reacting a compound corresponding to the following general formula:

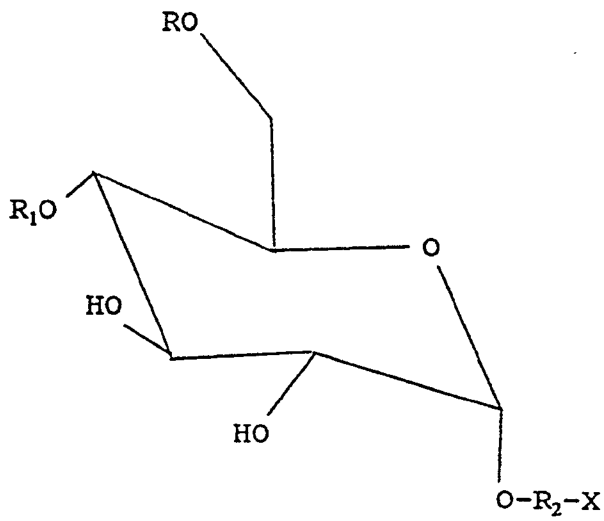

wherein X is as defined in claim 1; $R_2$ comprises from 2 to 7 glucose unites; and R and $R_1$ represent blocking groups; with an acid chloride or acid anhydride to form a corresponding acyl derivative;
(b) reacting the resulting compound with an acid or a base to remove the blocking groups R and $R_1$;
(c) contacting the resulting compound with a silylating agent to introduce a silyl radical at R, the silyl radical being substituted with (i) alkyl radicals selected from methyl, ethyl, n-propyl, isopropyl, n-butyl, isobutyl, t-butyl, n-pentyl and isomers thereof, n-hexyl, thexyldimethyl and cyclohexyl, or (ii) aryl radicals selected from phenyl, o-, p- or m-tolyl, o-, p- or m-methoxyphenyl, and di- and tri- methyl-substituted phenyl isomers; and
(d) contacting the resulting compound with an agent to cleave the acyl groups.

8. A process as claimed in claim 7 wherein the starting compound is 4,6-0-benzylidene-4-nitrophenyl-α-D-maltoheptaoside or 4,6-0-benzylidene-4-nitrophenyl-α-D-maltopentaoside.

9. A process as claimed in claim 7 or claim 8 wherein the acyl derivatives are acetyl or benzoyl.

10. A process as claimed in any of claims 7 to 9 wherein the silylating agent is selected from chlorosilane and chlorothexyldimethylsilane.

11. A process as claimed in any of claims 7 to 10 wherein the agent for cleaving the acyl groups is a sodium alkoxide which is present in a ratio of from about 0.05 to 2.5 equivalents of sodium alkoxide per acyl group in the acyl derivative; preferably sodium methoxide.

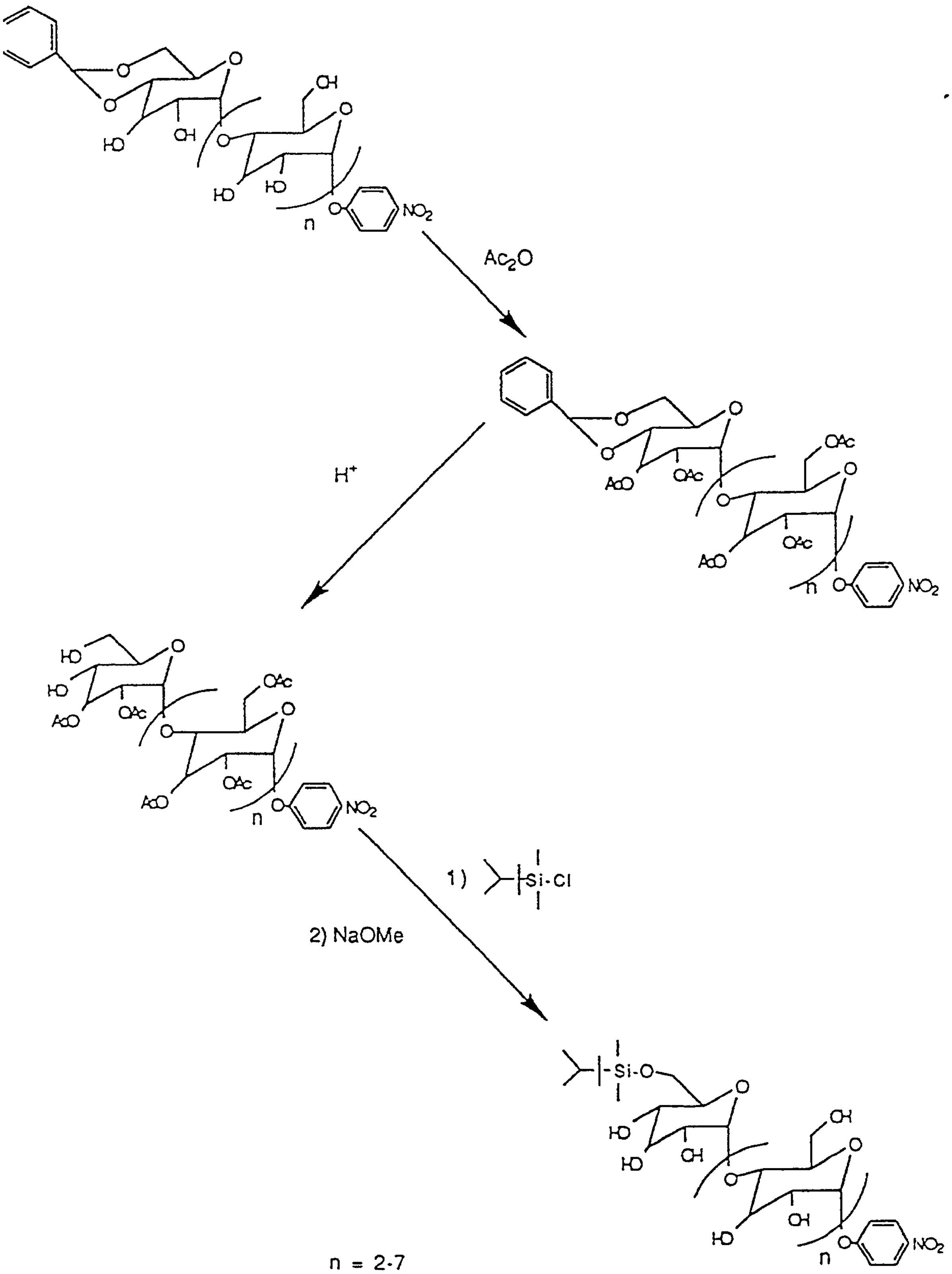


n = 2-7

FIGURE